(19) Europäisches Patentamt
European Patent Office
Office européen des brevets

(11) Veröffentlichungsnummer: **0 011 793**
**B1**

(12) # EUROPÄISCHE PATENTSCHRIFT

(45) Veröffentlichungstag der Patentschrift: **02.09.81**

(51) Int. Cl.³: **C 07 C 59/68,** C 07 C 59/70, C 07 C 51/235, C 07 C 59/66, C 07 C 51/265

(21) Anmeldenummer: **79104562.8**

(22) Anmeldetag: **19.11.79**

(54) **Verfahren zur Herstellung von Aryloxyessigsäuren.**

(30) Priorität: **30.11.78 DE 2851788**

(43) Veröffentlichungstag der Anmeldung: **11.06.80 Patentblatt 80/12**

(45) Bekanntmachung des Hinweises auf die Patenterteilung: **02.09.81 Patentblatt 81/35**

(84) Benannte Vertragsstaaten: **BE CH DE FR GB IT NL**

(56) Entgegenhaltungen:
**DE-A-2 620 254**
**FR-A-1 557 532**

(73) Patentinhaber: **BAYER AG, Zentralbereich Patente, Marken und Lizenzen, D-5090 Leverkusen 1, Bayerwerk (DE)**

(72) Erfinder: **Fiege, Helmut, Dr., Walter-Flex-Strasse 8, D-5090 Leverkusen 1 (DE)**
Erfinder: **Wedemeyer, Karlfried, Dr., Bilharzstrasse 7, D-5000 Köln 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 011 793 B1

Verfahren zur Herstellung von Aryloxyessigsäuren

Die Erfindung betrifft ein Verfahren zur Herstellung von Aryloxyessigsäuren durch Oxidation von Aryloxyäthanolen.

Aus Ullmann, Enzyklopädie der Technischen Chemie, 4. Auflage, Band 9, 578 (1975) ist bekannt, Aryloxyessigsäuren technisch durch Erhitzen der entsprechenden Aryloxyverbindungen (z. B. Phenole oder Naphthole) mit Monochloressigsäure in überschüssiger, wäßriger Natronlauge und anschließender Behandlung mit Salzsäure herzustellen. Nachteile der Verfahren sind u. a., daß die Chloressigsäure zum Ausgleich von Verlusten im Überschuß eingesetzt werden muß und das aus dem gesamten zur Chloressigsäure-Herstellung erforderlichen Chlor letztlich nur Natriumchlorid entsteht.

Auch die Oxidation von Aryloxyäthanol mit Sauerstoff oder Luft ist bereits mehrfach untersucht worden (SU 130 510, Zh. Prikl. Khim (Leningrad) 46, 2691 bis 2694), Kinetika i Kataliz 1 (1960), 125 bis 128 und Kinetika i Kataliz 2 (1961), 245). Die Ergebnisse sind jedoch bezüglich der Reaktionsbedingungen, der Ausbeute und der Katalysatorleistung unbefriedigend. Die bisher besten Ergebnisse werden bei der Oxidation mit Luft in wäßrig-alkalischem Medium an Platin-Aktivkohle-Katalysatoren mit 2,5 bis 10% Platin-Gehalt erzielt (Zh. Fiz. Khim 42 (1968), 266 bis 268). Die nach diesem Verfahren erreichten besten Phenoxyessigsäure-Ausbeuten betragen bei der Oxidation von Phenoxyäthanol 82% des theoretischen Umsatzes und bei der Oxidation von 2,4-Dichlorphenoxyäthanol 88% des theoretischen Umsatzes.

Voraussetzung für diese Ergebnisse sind sehr geringe Phenoxyäthanol-Konzentrationen im Einsatzgemisch (nur 2,5 Gew.-%), hohe Drucke (140 bar), Temperaturen von 140 bzw. 100° C sowie ein frischer Katalysator. Bei Wiederverwendung des Katalysators sinkt die Ausbeute auf 60% des theoretischen Umsatzes ab. Das nicht zur entsprechenden Phenoxyessigsäure umgesetzte Phenoxyäthanol geht durch oxidativen Abbau zu Kohlendioxid und dem Ausgangsphenol verloren.

Abgesehen von den technisch unvorteilhaften Bedingungen wie niedrige Einsatzkonzentration, Oxidation unter hohem Druck sowie der mangelnden Selektivität, besteht ein erheblicher Nachteil des Verfahrens in der geringen Leistungsfähigkeit (Aktivität) des Platin-Aktivkohle-Katalysators, der bei 2,5% bzw. 10% Platin-Gehalt nur 0,5 bzw. 0,6 kg Phenoxyessigsäure pro kg Katalysator und Stunde (entsprechend 20 bzw. 6 kg Phenoxyessigsäure pro kg Platin und Stunde) beträgt. Dies bedeutet, daß zur Erzielung kurzer Reaktionszeiten sehr große Mengen des wertvollen Platin-Katalysators (bezogen auf das Phenoxyäthanol) eingesetzt und dementsprechend hohe Handhabungsverluste in Kauf genommen werden müssen. Kleine Katalysatormengen haben sehr lange Reaktionszeiten zur Folge.

Es wurde ein Verfahren zur Herstellung von Aryloxyessigsäuren durch Oxidation von Aryloxyäthanolen der Formel

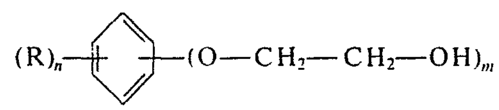

(I)

worin

m für 1 oder 2 steht,
n für die Zahl steht, die sich aus der Differenz (6 − m) ergibt, und
R entweder einzeln oder unabhängig voneinander für Wasserstoff, Alkyl, Cycloalkyl, Aryl, Aralkyl, Alkoxy, Cycloalkoxy, Aryloxy, Hydroxy, Halogen, Alkylcarbonyl, Arylcarbonyl, Carboxy, Nitro oder für einen an den Phenylring ankondensierten Benzolring oder Cycloalkanring stehen,

mit Sauerstoff oder Sauerstoff enthaltenden Gasen in wäßrig-alkalischen Medien bei Temperaturen von 0° C bis zum Siedepunkt des Reaktionsgemisches in Gegenwart von Platinmetall-Katalysatoren gefunden, dadurch gekennzeichnet, daß man die Oxidation in Gegenwart von Blei und/oder Wismut und/oder deren Verbindungen und gegebenenfalls zusätzlicher Gegenwart von Cadmium und/oder dessen Verbindungen durchführt.

Das erfindungsgemäße Verfahren kann am Beispiel der folgenden Reaktionsgleichung erläutert werden:

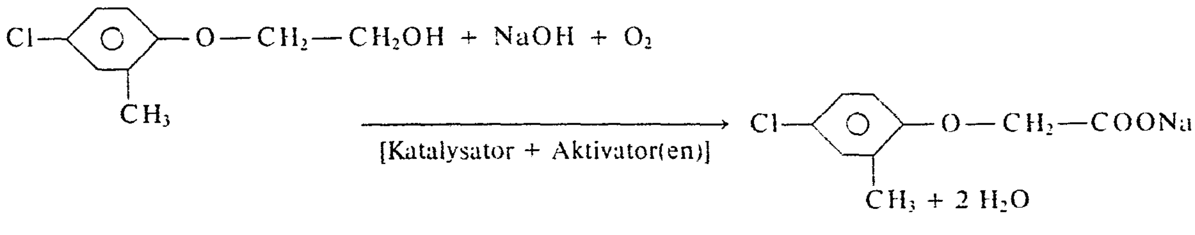


Alkylreste können geradkettige oder verzweigte Kohlenwasserstoffreste mit 1 bis 12, bevorzugt 1 bis 6, Kohlenstoffatomen sein. Bevorzugte Alkylreste für das erfindungsgemäße Verfahren sind

Niederalkylreste. Als Alkylreste seien beispielsweise genannt: Methyl, Äthyl, Propyl, Isopropyl, Butyl, Isobutyl, sec-Butyl, tert.-Butyl, Pentyl, Isopentyl, tert. Amyl, Hexyl, Isohexyl, Heptyl, Isoheptyl, tert.-Octyl, Isooctyl, Nonyl, Isononyl, Decyl, Isodecyl, Undecyl, Isoundecyl, Dodecyl und Isododecyl.

Cycloalkylreste können cyclische Kohlenwasserstoffreste mit 4 bis 9, bevorzugt 5 und 6, Kohlenstoffatome sein. Beispielsweise seien der Cyclopentyl- und der Cyclohexyl-Rest genannt.

Als Arylreste für das erfindungsgemäße Verfahren seien bevorzugt der Phenyl- und der Naphthyl-Rest genannt.

Aralkylreste können Alkylreste mit 1 bis 6 Kohlenstoffatomen, bevorzugt Niederalkylreste, sein, die durch einen aromatischen Kohlenwasserstoffrest mit 6 bis 12 Kohlenstoffatomen, bevorzugt Phenyl und Naphthyl, substituiert sind. Benzyl-, $\alpha$-Methyl-benzyl-, $\alpha,\alpha$-Dimethyl-benzyl-Gruppen seien als Beispiel genannt.

Alkoxyreste können im aliphatischen Teil aus bis 12, bevorzugt aus bis zu 6, Kohlenstoffatomen bestehen. Besonders bevorzugt wird ein niederer Alkoxyrest. Als Alkoxyreste seien beispielsweise genannt: Methoxy, Äthoxy, Propoxy, Isopropoxy, Butoxy, Isobutoxy, tert.-butoxy, Pentoxy, Isopentoxy, Hexoxy und Isohexoxy, Methylendioxy.

Als Cycloalkoxyreste seien bevorzugt der Cyclopentoxy- und der Cyclohexoxyrest genannt.

Als Aryloxyreste seien bevorzugt der Phenoxy und der Naphtoxyrest genannt.

Halogene können Fluor, Chlor, Brom und Jod, bevorzugt Chlor und Brom, sein.

Als Alkylcarbonylreste seien bevorzugt Niederalkylcarbonylreste ($C_1$ bis $C_6$), wie der Acetylrest, genannt.

Als Arylcarbonylreste sei bevorzugt der Benzoylrest genannt.

Durch Ankondensation eines Benzolrings an den Phenylring kann beispielsweise das Naphthalinringsystem entstehen.

Durch Ankondensation eines Cycloalkanrings an den Phenylring kann beispielsweise das Tetralinringsystem entstehen.

Es ist selbstverständlich möglich, daß die o. g. Substituenten durch übliche, unter den Reaktionsbedingungen inerte Reste substituiert sein können. Beispielsweise seien genannt: Fluor, Chlor, Methyl, Methoxy.

Besonders bevorzugt für das erfindungsgemäße Verfahren werden Aryloxyäthanole in denen m gleich 1 und n 1, 2 oder 3 ist.

Insbesondere bevorzugt für das erfindungsgemäße Verfahren werden Aryloxyäthanole der Formel

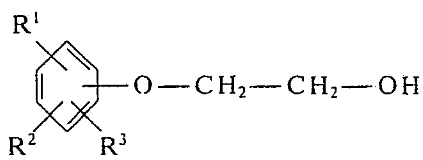

worin

$R^1$, $R^2$, $R^3$ gleich oder verschieden sind und für Wasserstoff, Niederalkyl, Niederalkoxy, Phenyl, Naphthyl, Phenoxy, Niederalkylcarbonyl, Fluor und Chlor stehen.

Die Herstellung der Aryloxyäthanole ist an sich bekannt.

Sie können beispielsweise hergestellt werden, indem man Äthylenoxid an die Hydroxygruppe(n) eines entsprechend substituierten Phenols oder Naphthols addiert (Monatshefte Chemie 77, [1947] 80 bis 85).

Im einzelnen seien die folgenden Aryloxyäthanole genannt:

Phenoxyäthanol, 2-Methyl-phenoxyäthanol, 3-Methyl-phenoxyäthanol, 4-Methyl-phenoxyäthanol, 2,3-Dimethyl-phenoxyäthanol, 2,4-Dimethyl-phenoxyäthanol, 2,5-Dimethyl-phenoxyäthanol, 2,6-Dimethyl-phenoxyäthanol, 3,4-Dimethyl-phenoxyäthanol, 3,5-Dimethyl-phenoxyäthanol, 2-Chlor-phenoxyäthanol, 3-Chlor-phenoxyäthanol, 4-Chlor-phenoxyäthanol, 2-Chlor-4-methyl-phenoxyäthanol, 2-Chlor-5-methyl-phenoxyäthanol, 2-Chlor-6-methyl-phenoxyäthanol, 4-Chlor-2-methyl-phenoxyäthanol, 4-Chlor-3-methyl-phenoxyäthanol, 2-Chlor-4-fluor-phenoxyäthanol, 2,3-Dichlor-phenoxyäthanol, 2,4-Dichlor-phenoxyäthanol, 2,5-Dichlor-phenoxyäthanol, 2,6-Dichlor-phenoxyäthanol, 3,4-Dichlor-phenoxyäthanol, 3,5-Dichlor-phenoxyäthanol, 4,6-Dichlor-2-methyl-phenoxyäthanol, 2,6-Dichlor-4-methyl-phenoxyäthanol, 2,6-Dichlor-3-methyl-phenoxyäthanol, 2,4-Dimethyl-6-chlor-phenoxyäthanol, 2,6-Dimethyl-4-chlor-phenoxyäthanol, 3,5-Dimethyl-4-Chlor-phenoxyäthanol, 2,4,5-Trichlor-pehnoxyäthanol, 2,4,6-Trichlor-phenoxyäthanol, 3,4,5-Trichlor-phenoxyäthanol, 2,3,4-Trichlor-phenoxyäthanol, 4-Nonyl-phenoxyäthanol, $\alpha$-Naphthoxyäthanol und $\beta$-Naphthoxyäthanol. Bevorzugte Aryloxyäthanole für das erfindungsgemäße Verfahren sind Phenoxyäthanol, 4-Chlor-2-methyl-phenoxyäthanol, 2,4-Dichlor-phenoxyäthanol und 2,4,5-Trichlor-phenoxyäthanol.

Nach dem erfindungsgemäßen Verfahren wird die Oxidation der Aryloxyäthanole zu den Aryloxyessigsäuren in Gegenwart von Aktivatoren aus Blei und/oder Wismut und/oder deren Verbindungen und gegebenenfalls zusätzlicher Gegenwart von Cadmium und/oder dessen Verbindungen durchgeführt.

Die mit der Erfindung erzielten Vorteile bestehen darin, daß durch die Gegenwart der erfindungsgemäß zu verwendenden Metalle die Selektivität, die Aktivität und die Wiederverwendbarkeit des Katalysators ganz erheblich gesteigert werden. Dadurch können sehr hohe Ausbeuten erzielt, die auf Aryloxyäthanol bezogene Katalysatormenge erheblich reduziert, der Arbeitsdruck erheblich vermindert und auch die Einsatzkonzentrationen der Aryloxyäthanole deutlich erhöht werden.

Die Platinmetallkatalysatoren sind für das erfindungsgemäße Verfahren ohne die Gegenwart der Aktivatoren völlig inaktiv. Erkennbar aktiv werden sie erst nach dem erfindungsgemäßen Zusatz von Blei oder Wismut. Durch die erfindungsgemäße zusätzliche Anwesenheit von Cadmium werden sowohl die Aktivität als auch die Selektivität des Katalysators noch weiter gesteigert.

Die Mengen, in denen die erfindungsgemäß zu verwendenden Aktivatoren eingesetzt werden, können in weiten Grenzen schwanken. Die Aktivatorwirkung macht sich bereits bei Zusätzen von $5 \times 10^{-6}$ Mol Metall bzw. Metallverbindung je Mol Aryloxyäthanol deutlich bemerkbar. Es können auch 0,1 Mol oder mehr Aktivator je Mol Aryloxyäthanol eingesetzt werden, jedoch bieten diese hohen Zusätze im allgemeinen keinen Vorteil. Im allgemeinen haben sich Zusätze von $1 \times 10^{-5}$ bis $1 \times 10^{-1}$ Mol, vorzugsweise $5 \times 10^{-5}$ bis $5 \times 10^{-2}$ Mol, Metall bzw. Metallverbindung je Mol zu oxidierendes Aryloxyäthanol bewährt.

Die erfindungsgemäß als Aktivatoren zu verwendenden Metalle können in elementarer Form und/oder in Form ihrer Verbindungen, z. B. als Oxide, Hydroxide, Salze von Wasserstoffsäuren wie Chloride, Bromide, Jodide, Sulfide, Selenide, Teluride, oder als Salze von anorganischen Sauerstoffsäuren, wie Nitrate, Nitrite, Phosphite, Phosphate, Carbonate, Perchlorate, Antimonate, Arseniate, Selenite, Seleniate, Borate, oder als Salze von Sauerstoffsäuren der Übergangsmetalle, wie Vanadate, Niobate, Tantalate, Chromate, Molybdate, Wolframate, Permanganate, oder als Salze organischer aliphatischer oder aromatischer Säuren, wie z. B. Formiate, Acetate, Propionate, Benzoate, Salicylate, Lactate, Aryloxyacetate, Zitrate oder als Phenolate usw. eingesetzt werden.

Die Aktivatoren können im Reaktionsgemisch jeweils löslich, teilweise löslich oder unlöslich sein.

Auch Kombinationen dieser Aktivatoren untereinander und/oder mit anderen, nicht als Aktivatoren genannten Elemente der Verbindungen können eingesetzt werden.

Die Aktivatoren für das erfindungsgemäße Verfahren können in unterschiedlichen und auch gemischten Wertigkeitsstufen vorliegen; auch können Änderungen in der Wertigkeit während der Reaktion eintreten. Sofern die Aktivatoren nicht bereits als Oxyde und/oder Hydroxyde zugegeben werden, ist es möglich, daß sie sich im alkalischen Medium ganz oder teilweise in diese umwandeln. Nach der Reaktion kann der Platinmetall-Katalysator mit dem schwer löslichen Aktivator abfiltriert und in weiteren Oxidationsreaktionen wiederverwendet werden. Verluste an Platinmetall-Katalysatoren und/oder Aktivatoren sind gegebenenfalls zu ersetzen.

Der Aktivator kann als Feststoff, vorzugsweise in feinverteilter Form, oder in gelöster Form, den Reaktionskomponenten zugesetzt werden. Man kann den Aktivator auch schon bei der Herstellung des Platinmetall-Katalysators zugeben oder den Platinmetall-Katalysator mit dem Aktivator imprägnieren. Der Aktivator kann auch als Trägermaterial für das Platinmetall eingesetzt werden.

Unter Platinmetallen, die für das erfindungsgemäße Verfahren als Katalysatoren eingesetzt werden können, sind die chemisch nahe verwandten, in der Natur meist gemeinsam auftretenden Metalle Platin, Palladium, Rhodium, Iridium, Ruthenium und Osmium zu verstehen. Bevorzugt werden Platin oder Palladium als Katalysatoren eingesetzt.

Die als Katalysatoren verwendeten Platinmetalle können den Reaktionskomponenten in verschiedenster Form zugegeben werden, beispielsweise in elementarer Form, z. B. als sogenanntes Mohr, in Kombination mit anderen Platinmetallen oder in Form von Verbindungen, z. B. als Oxide oder in Form anderer Verbindungen.

Die Platinmetalle können auch auf einen Träger aufgebracht sein. Als Träger seien beispielsweise Aktivkohle, Graphit, Kieselgur, Kieselgel, Spinelle, Aluminiumoxid, Asbest, Calciumcarbonat, Magnesiumcarbonat, Bariumsulfat oder auch organische Trägermaterialien genannt.

Besonders bevorzugt als Träger sind pulverförmige Aktivkohlen, beispielsweise pulverförmige sogenannte Medizinalkohle oder aus Holz hergestellte pulverförmige Aktivkohlen wie sie für Entfärbungszwecke verwendet werden.

Der Platinmetallgehalt der Trägerkatalysatoren kann in weiten Grenzen schwanken. Besonders bewährt haben sich Trägerkatalysatoren mit einem Platinmetallgehalt unter 25 Gew.-%. Insbesondere bevorzugt werden palladiumhaltige Trägerkatalysatoren mit einem Palladiumgehalt von 1 bis 20 Gew.-% und platinhaltige Trägerkatalysatoren mit einem Platingehalt von 0,1 bis 5 Gew.-%.

Die Mengen, in denen die Platinmetall-Katalysatoren — bezogen auf das Aryloxyäthanol — verwendet werden, können in weiten Grenzen schwanken. Die Mengen hängen u. a. von der gewünschten Oxidationsgeschwindigkeit ab. Im allgemeinen ist ihre Menge geringer als die Aryloxyäthanolmenge im Einsatzgemisch. Im allgemeinen verwendet man Mengen unter 30 Gew.-%, insbesondere Mengen von 0,5 bis 20 Gew.-%, bezogen auf das Aryloxyäthanol.

Das erfindungsgemäße Verfahren kann üblicherweise so durchgeführt werden, daß man Sauerstoff oder Sauerstoff enthaltende Gase wie Luft mit der das alkalische Mittel, den Platinmetall-Katalysator und den Aktivator (evtl. mehrere) enthaltenden wäßrigen Lösung (bzw. Suspension) des Aryloxyäthanols in guten Kontakt bringt. Im allgemeinen führt man die Umsetzung bei

Atmosphärendurck (1 bar) durch, jedoch kann auch bei höheren oder niedrigen Drucken oxidiert werden. Im allgemeinen führt man das erfindungsgemäße Verfahren im Druckbereich von 0,5—10 bar durch.

Der Verlauf der Reaktion kann über die aufgenommene Sauerstoffmenge verfolgt werden und wird abgebrochen, wenn die für die gewünschte Aryloxyessigsäure theoretisch erforderliche Sauerstoffmenge aufgenommen ist. Meist hört die Sauerstoffaufnahme in diesem Stadium von selbst auf oder sie verlangsamt sich. Der Fortgang der Reaktion kann auch auf andere Weise, z. B. durch Bestimmung des verbrauchten Aryloxyäthanols oder der gebildeten Aryloxyessigsäure verfolgt werden.

Zur Aufarbeitung werden der Platinmetall-Katalysator und der ungelöste Aktivator abgetrennt, beispielsweise durch Filtration. Die erhaltenen Alkalisalzlösungen der Aryloxyessigsäure werden dann nach bekannten Methoden aufgearbeitet (Ullmann, Enzyklopädie der Technischen Chemie, 4. Auflage, Band 9, Seiten 578 bis 580).

Die Reihenfolge, in der der Platinmetallkatalysator, der Aktivator, das wäßrige Alkali und das Aryloxyäthanol zusammengegeben werden, ist beliebig. So können der Platinmetallkatalysator und der Aktivator der Mischung bzw. Lösung aus wäßrigem Alkali und Aryloxyäthanol zugesetzt werden; man kann auch den Platinmetallkatalysator und den Aktivator (oder die Aktivatoren) vorlegen und die Mischung aus wäßrigem Alkali und Aryloxyäthanol zusetzen; schließlich ist es auch möglich, den Platinmetallkatalysator, einen Teil des wäßrigen Alkalis und den Aktivator (oder die Aktivatoren) vorzulegen und das Aryloxyäthanol dann zusammen mit dem restlichen Alkali zuzusetzen. Ferner ist es auch möglich, den Aktivator (oder die Aktivatoren) der Mischung der Reaktionskomponente zuzusetzen.

Die Konzentration des Aryloxyäthanols in der wäßrig-alkalischen Reaktionsmischung wird im allgemeinen so gewählt, daß die entstehende Aryloxyessigsäure während der Reaktion gelöst vorliegt. Zweckmäßig sind Konzentrationen von 2 bis 25 Gew.-%. Gegebenenfalls kann die Löslichkeit durch den Zusatz von inerten Lösungsmitteln bzw. Lösungsvermittlern verbessert werden.

Es ist auch möglich, Gemische verschiedener Aryloxyäthanole zu oxidieren.

Die Oxidation nach dem erfindungsgemäßen Verfahren erfolgt vorzugsweise in Gegenwart von Alkalien, z. B. von Natrium- oder Kaliumhydroxyd. Als Alkali wird dabei so bemessen, daß auf ein Mol der entstehenden Carboxylgruppe 0,5 bis 10, vorzugsweise 0,8 bis 8, insbesondere 1 bis 6 Mol Alkali kommen. Die im Einzelfall günstigste Alkalimenge hängt u. a. vom Platinmetall, dem Aryloxyäthanol, dem Aktivator bzw. der Aktivatorkombination ab und kann in Vorversuchen leicht ermittelt werden.

Die für das erfindungsgemäße Verfahren mögliche Reaktionstemperatur kann zwischen dem Erstarrungspunkt und dem Siedepunkt des Reaktionsgemisches liegen.

Die im Einzelfall anzuwendende Reaktionstemperatur richtet sich unter anderem nach dem Katalysatorsystem, der Alkalikonzentration, den Substanzeigenschaften der Edukte und Produkte und den technischen Gegebenheiten (gewünschte Reaktionsgeschwindigkeit, Wärmeabfuhr). Bevorzugt für das erfindungsgemäße Verfahren ist der Temperaturbereich von etwa 20 bis 100° C.

Nach dem erfindungsgemäßen Verfahren können Aryloxyessigsäuren der Formel

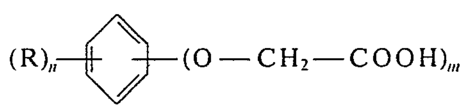

worin

m und n die obengenannte Bedeutung haben,

hergestellt werden.

Aryloxyessigsäuren sind wertvolle organische Zwischenprodukte und als Herbizide von großer Bedeutung (Chemie der Pflanzenschutz- und Schädlingsbekämpfungsmittel, Band 2, Seite 273 bis 278 [1970], Römpps Chemie-Lexikon, 7. Aufl. Bd. 4, S. 2623 [1974]).

Beispiel 1

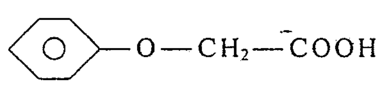

In einem mit Rührer, Innenthermometer und Gaszuleitung versehenen, über einen Außenmantel thermostatisierbaren Reaktionsgefäß werden 0,65 g palladiumhaltige Aktivkohle (sog. Medicinalkohle mit 5 Gew.-% Palladiumgehalt), 0,025 g festes $Bi(NO_3)_3 \cdot 5\ H_2O$ (entsprechend einer Wismut-Menge von $5,2 \cdot 10^{-5}$ Mol), 100 ml 1,1 n-Natronlauge und 13,8 g (0,1 Mol) Phenoxyäthanol eingebracht.

Nach Verdrängen der Luft aus dem Reaktionsgefäß durch Sauerstoff wird der Rührer angestellt, das Reaktionsgemisch auf 90° C gebracht und bei dieser Temperatur reiner Sauerstoff unter Normaldruck

in die Mischung eingeleitet. Nach 1,5 Stunden und Aufnahme von 0,1 Mol Sauerstoff kommt die Oxidation zum Stillstand.

Nach Abfiltrieren des Kontaktes von der noch warmen Lösung und Nachwaschen mit etwas Wasser wurde das Filtrat zur Bestimmung der »Neutralstoffe« (Phenol und nicht umgesetztes Phenoxyäthanol) mit 20%iger Schwefelsäure auf pH 6,5 eingestellt, mit Äther extrahiert und die vereinigten Ätherextrakte nach Trocknen über $Na_2SO_4$ eingedampft. Der »Rückstand aus der pH-6,5-Phase« war in diesem Fall vernachlässigbar gering. Zur Bestimmung der Phenoxyessigsäure wurde die wäßrige Phase (das Filtrat) anschließend mit 20%iger $H_2SO_4$ auf pH 1 angesäuert, wie vorstehend extrahiert und eingedampft. Der »Rückstand aus der pH-1-Phase« bestand im vorliegenden Fall aus 15,2 g weißer, kristalliner Phenoxyessigsäure, die nach der acidimetrischen Bestimmung >99%ig war und einen Fp von 98,5 bis 99° C aufwies. Die Ausbeute beträgt mithin praktisch 100% der Theorie.

Diese Art der Aufarbeitung hatte lediglich analytische Gründe. Man kann z. B. auch das in der Kälte schwerlösliche Natriumsalz der Phenoxyessigsäure direkt nach Abkühlen des Filtrats absaugen oder die in Wasser schwerlösliche Phenoxyessigsäure direkt durch Ansäuern freisetzen und abtrennen.

Der wie vorstehend beschrieben abgetrennte Katalysator wird beim nächsten Ansatz wiederverwendet usw. Er erbrachte auch bei der 33. Wiederverwendung noch die gleiche Ausbeute an Phenoxyessigsäure (100% der Theorie). Die Versuche wurden dann abgebrochen.

## Beispiel 2

Es wird wie in Beispiel 1 gearbeitet, jedoch mit dem Unterschied, daß anstelle von Wismut $5 \cdot 10^{-4}$ Mol Blei in Form einer 0,1 m $Pb(NO_3)_2$-Lösung zum Reaktionsgemisch gegeben werden.

In diesem Fall kommt die Sauerstoffaufnahme nach 1,75 Stunden nach Aufnahme von etwa 0,1 Mol $O_2$ zum Stillstand. Die Aufarbeitung ergibt ca. 1 g »Neutralstoffe« aus der »pH 6,5-Phase« und ca. 14,1 g >99%ige Phenoxyessigsäure vom Fp 98,5 bis 99° C aus der »pH 1-Phase«. Die Ausbeute beträgt mithin 93% der Theorie.

## Beispiel 3

(Vergleichsbeispiel)

Es wird wie in Beispiel 1 gearbeitet, jedoch mit dem Unterschied, daß weder Blei noch Wismut als Aktivator zum Reaktionsgemisch gegeben werden.

In diesem Fall erfordert die Aufnahme von 0,1 Mol $O_2$ ca. 10 Stunden, wobei die Sauerstoffaufnahme nach dieser Zeit noch nicht beendet ist.

Arbeitet man nach Aufnahme der stöchiometrischen $O_2$-Menge auf, so ergibt die Aufarbeitung der »pH 6,5-Phase« 6,7 g »Neutralstoffe«, die laut GC vorwiegend aus Phenol bestehen und die Aufarbeitung der »pH 1-Phase« nur 6,3 g einer verunreinigten Phenoxyessigsäure vom Fp 93,5 bis 96° C (Ausbeute ca. 40% der Theorie).

Das Beispiel 3 zeigt, daß ohne den Zusatz der erfindungsgemäßen Aktivatoren die Reaktionszeit an Palladium deutlich verlängert, die Ausbeute durch oxidativen Abbau zu Phenol stark vermindert und die Qualität der erhaltenen Phenoxyessigsäure verschlechtert wird.

## Beispiele 4 bis 11

Es wird wie in Beispiel 1 gearbeitet, jedoch mit dem Unterschied, daß die Aktivatormenge, Natronlaugekonzentration, Phenoxyäthanolmenge und/oder die Temperatur verändert sind. Das eingesetzte Natronlauge-Volumen, die Katalysatormenge und die Aktivatorart bleiben konstant. Die Bedingungen und Ergebnisse sind in Tabelle 1 zusammengestellt.

Tabelle 1

Oxidation von Phenoxyäthanol an 0,65 g palladiumhaltiger Aktivkohle (5% Palladium auf Medicinalkohle) in Gegewart von Wismut als Aktivator in 100 ml Natronlauge unter Normaldruck mit Sauerstoff bis zur Aufnahme von 1 mol $O_2$ pro mol Phenoxyäthanol

| Beisp. Nr. | Phenoxy-äthanol Menge | Normalität der NaOH | Aktivator-menge $(Bi(NO_3)_3)$ | Temp. | Zeit für $O_2$-Aufnahme | Phenoxyessigsäure pH 1-Phase Menge | Fp | Ausbeute |
|---|---|---|---|---|---|---|---|---|
| | g | mol/l | mol | °C | Stdn. | g | °C | % d. Th. |
| 4 | 13,8 | 4 | $1\times10^{-5}$ | 70 | 11 | 13,6 | 98,5–99 | 90 |
| 5 | 13,8 | 4 | $1\times10^{-4}$ | 70 | 4 | 15 | 98.5–99 | 99 |
| 6 | 13,8 | 4 | $1\times10^{-3}$ | 70 | 4,5 | 14,9 | 98,5–99 | 98 |
| 7 | 13,8 | 2 | $5\times10^{-5}$ | 90 | 2 | 15,2 | 98,5–99 | 100 |
| 8 | 13,8 | 4 | $5\times10^{-5}$ | 95 | 3,3 | 15,2 | 98,5–99 | 100 |
| 9 | 17,25 | 1,4 | $5\times10^{-5}$ | 90 | 2,3 | 19 | 98,5–99 | 100 |
| 10 | 20,7 | 1,7 | $5\times10^{-5}$ | 90 | 9,8 | 22,8 | 98,5–99 | 100 |
| 11 | 27,6 | 2,2 | $5\times10^{-5}$ | 90 | 22,7 | 30 | 98,5–99 | 99 |

Beispiele 12 bis 17

Apparatur und Arbeitsweise entsprechen dem Beispiel 1. In das Oxidationsgefäß eingebracht werden 0,65 g platinhaltige Aktivkohle (sog. Entfärbekohle mit 1 Gew.-% Platingehalt), 100 ml 5 n-Natronlauge, 13,8 g (0,1 Mol) Phenoxyäthanol, 0,0005 Mol (Blei(II)-nitrat (als 0,5 m Lösung) oder 0,0005 Mol Wismut(III)-nitrat (als $Bi(NO_3)_3 \cdot 5\,H_2O$) als Aktivator sowie gegebenenfalls 0,0001 Mol Cadmium(II)-nitrat (in Form einer 0,1 m Lösung) als zusätzlichem Aktivator (Beispiele 14 und 15).

Beim Vergleichsbeispiel 16 wird völlig ohne Aktivatoren gearbeitet, beim Vergleichsbeispiel 17 nur Cadmium zugesetzt.

Nach Verdrängen der Luft aus dem Oxidationsgemäß wird auf 70°C erwärmt und bei dieser Temperatur unter Normaldruck Sauerstoff in das Reaktionsgemisch eingerührt. Die Ergebnisse sind in Tabelle 2 zusammengestellt.

Tabelle 2

Einfluß von Blei-, Wismut- und Cadmium-Zusätzen auf die Oxidation von 0,1 mol Phenoxyäthanol an platinhaltiger Aktivkohle (Bedingungen s. Text) in 100 ml 5 n Natronlauge

| Beisp. Nr. | Aktivator | Zusatz-Aktivator | $O_2$-Aufnahme | | Produkte | | |
|---|---|---|---|---|---|---|---|
| | | | | | Phenol[a]) | Phenoxyessigsäure | |
| | | | Menge | Zeit | Menge | Menge[b]) | Ausbeute |
| | | | mol | Stdn. | g | g | % d. Th. |
| 12 | Pb | ohne | 0,1 | 6 | 5,6 | 5,2 | 34 |
| 13 | Bi | ohne | 0,1 | 6 | 5,3 | 5,3 | 35 |
| 14 | Pb | Cd | 0,1 | 3,5 | 0,3 | 14,9 | 98 |
| 15 | Bi | Cd | 0,11 | 6 | 4,1 | 8,1 | 53 |
| 16 | ohne | ohne | 0,00 | 6 | Phenoxyäthanol praktisch unverändert zurückgewonnen | | 0 |
| 17 | ohne | Cd | 0,00 | 6 | | | 0 |

a) Extrakt-Rückstand »pH 6,5-Phase«; Hauptkomponente Phenol, daneben nicht umgesetztes Phenoxyäthanol.
b) Extrakt-Rückstand »pH 1-Phase«.

Wie das Vergleichsbeispiel 16 in Tabelle 2 zeigt, wird Phenoxyäthanol unter den gewählten Bedingungen an dem nur 1% Platin enthaltenden Kontakt praktisch nicht oxidiert. Eine Oxidation tritt erst ein, wenn Blei oder Wismut im Reaktionsgemisch anwesend sind (Beispiele 12 und 13). Eine weitere Verbesserung der Oxidationsergebnisse tritt ein, wenn zusätzlich noch Cadmium im Reaktionsgemisch anwesend ist (Beispiele 14 und 15). Cadmium allein wirkt noch nicht aktivierend auf die platinhaltige Aktivkohle (Vergleichsbeispiel 17).

Beispiele 18 bis 19

Wird wie im Beispiel 14 gearbeitet, jedoch nicht bei 70° C, sondern bei 50 oder 60° C, so betragen die Reaktionszeiten 5 bzw. 3 Stunden und die Phenoxyessigsäure-Ausbeuten in beiden Fällen 100% der Theorie (Fp. 98,5 bis 99° C).

Beispiel 20

Wird wie im Beispiel 19 bei 60° C gearbeitet, jedoch mit dem Unterschied, daß die $5 \cdot 10^{-4}$ Mol Blei nicht in Form einer Blei(II)-nitrat-Lösung in die Reaktionsmischung eingebracht werden, sondern in Form von feingepulvertem

a) Bleimetall
b) Blei(II)-oxid
c) Blei(II)-sulfat
d) Blei(II)-acetat
e) Blei(II,IV)-oxid (Mennige)

zugesetzt werden, so beträgt die Reaktionszeit in allen Fällen wiederum etwa 3 Stunden und die Phenoxyessigsäure-Ausbeute praktisch 100% der Theorie.

Beispiele 21 bis 31

Es wird wie in Beispiel 1 gearbeitet. Oxidiert werden jeweils 0,05 Mol Aryloxyäthanol in 100 ml 5gew.-%iger Natronlauge bei 90°C mit reinem Sauerstoff unter Normaldruck an 0,65 g palladiumhaltiger Aktivkohle (Medicinalkohle) mit 10 Gew.-% Palladiumgehalt in Gegenwart von $5 \cdot 10^{-5}$ Mol Wismut(III)-nitrat.

In Tabelle 3 sind die mit verschiedenen Aryloxyäthanolen erhielten Ergebnisse zusammengestellt. Da stets die gleichen Reaktionsbedingungen angewandt wurden, sind die Ergebnisse nicht unbedingt optimal.

Tabelle 3

Oxidation verschiedener Aryloxyäthanole (Bedingungen s. Text)

| Beisp. Nr. | Aryloxyäthanol Bezeichnung | Einsatz g | Zeit für Aufnahme von 0,05 Mol $O_2$ | Aryloxyessigsäure Bezeichnung | Ausbeute g[a]) | Ausbeute % d. Th. |
|---|---|---|---|---|---|---|
| 21 | 4-Methyl-phenoxyäthanol | 7,6 | 0,75[c]) | 4-Methyl-phenoxyessigsäure | 8,2 | 99 |
| 22 | 3,4-Dimethyl-phenoxyäthanol | 8,3 | 1,0 | 3,4-Dimethyl-phenoxyessigsäure | 8,6 | 95 |
| 23 | 4-tert.-Butyl-phenoxyäthanol | 9,7 | 0,75 | 4-tert.-Butyl-phenoxyessigsäure | 10,3 | 98 |
| 24 | 4-Chlor-phenoxyäthanol | 8,6 | 1,25 | 4-Chlorphenoxyessigsäure | 8,7 | 94 |
| 25 | 4-Chlor-2-methyl-phenoxyäthanol | 9,3 | 2,0 | 4-Chlor-2-methyl-phenoxyessigsäure | 9,8 | 98 |
| 26 | 4-Chlor-3,5-dimethyl-phenoxyäthanol | 10,0 | 1,0 | 4-Chlor-3,5-dimethyl-phenoxyessigsäure | 9,9 | 92 |
| 27 | 2,4-Dichlor-phenoxyäthanol | 10,3 | 1,75 | 2,4-Dichlor-phenoxyessigsäure | 10,5 | 95 |
| 28 | 2,4,5-Trichlor-phenoxyäthanol | 12,1 | 1,5 | 2,4,5-Trichlor-phenoxyessigsäure | 12,3 | 96 |
| 29 | 4-Nitro-phenoxyäthanol | 9,1 | 4,5 | 4-Nitro-phenoxyessigsäure | 3,8[b]) | 33 |
| 30 | 4-Methoxy-phenoxyäthanol | 8,4 | 1,0 | 4-Methoxy-phenoxyessigsäure | 8,8 | 97 |
| 31 | β-Naphthoxyäthanol | 9,4 | 2,5 | β-Naphthoxyessigsäure | | |

[a]) Nur »pH 1-Phase«, nach titrimetrischer Bestimmung praktisch immer 99 bis 100%ig.
[b]) Nur ca. 85%ig.
[c]) An 0,65 g Aktivkohle mit 5% Palladium-Gehalt doppelte Reaktionszeit, aber gleiche Ausbeute (99% der Theorie).

**Patentansprüche**

1. Verfahren zur Herstellung von Aryloxyessigsäuren durch Oxidation von Aryloxyäthanolen der Formel

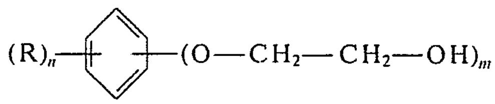

(I)

in der

m für 1 oder 2 steht,
n für die Zahl steht, die sich aus der Differenz (6 — m) ergibt, und
R entweder einzeln und unabhängig voneinander für Wasserstoff, Alkyl, Cycloalkyl, Aryl, Aralkyl, Alkoxy, Cycloalkoxy, Aryloxy, Hydroxy, Halogen, Alkylcarbonyl, Arylcarbonyl, Carboxy, Nitro oder für einen an den Phenylring ankondensierten Benzolring oder Cycloalkanring stehen,

mit Sauerstoff oder Sauerstoff enthaltenden Gasen in wäßrig alkalischen Medien bei Temperaturen von 0°C bis zum Siedepunkt des Reaktionsgemisches in Gegenwart von Platinmetall-Katalysatoren, dadurch gekennzeichnet, daß man die Oxidation in Gegenwart von Aktivatoren aus Blei und/oder Wismut und/oder deren Verbindungen und gegebenenfalls zusätzlicher Gegenwart von Cadmium und/oder dessen Verbindungen vornimmt.

2. Verfahren nach Anspruch 1, dadurch gekennzeichnet, daß man Blei und/oder Wismut und gegebenenfalls zusätzlich Cadmium in Mengen von $1 \cdot 10^{-5}$ bis $1 \cdot 10^{-1}$ Mol Metall bzw. Metallverbindung je Mol zu oxidierendes Aryloxyäthanol einsetzt.

3. Verfahren nach Anspruch 1, dadurch gekennzeichnet, daß man Blei und/oder Wismut und gegebenenfalls zusätzlich Cadmium in Mengen von $5 \cdot 10^{-5}$ bis $5 \cdot 10^{-2}$ Mol Metall bzw. Metallverbindung je Mol zu oxidierendes Aryloxyäthanol einsetzt.

4. Verfahren nach Anspruch 1 bis 3, dadurch gekennzeichnet, daß man als Platinmetall-Katalysatoren Platin und/oder Palladium verwendet.

5. Verfahren nach Anspruch 1 bis 4, dadurch gekennzeichnet, daß man Blei und/oder Wismut und gegebenenfalls zusätzlich Cadmium in Kombination mit Platinkatalysatoren verwendet.

6. Verfahren nach Anspruch 1 bis 4, dadurch gekennzeichnet, daß man Blei und/oder Wismut in Kombination mit Palladiumkatalysatoren verwendet.

7. Verfahren nach Anspruch 1 bis 6, dadurch gekennzeichnet, daß das Platinmetall auf einem Träger aufgebracht ist.

8. Verfahren nach Anspruch 1 bis 7, dadurch gekennzeichnet, daß man als Träger für das Platinmetall Aktivkohle verwendet.

9. Verfahren nach Anspruch 1 bis 8, dadurch gekennzeichnet, daß der Platinmetallgehalt der Trägerkatalysatoren unter 25 Gew.-% liegt.

10. Verfahren nach Anspruch 1 bis 8, dadurch gekennzeichnet, daß der Platinmetallgehalt der Trägerkatalysatoren bei Verwendung von Palladium unter 20 Gew.-% liegt und bei Verwendung von Platin unter 5 Gew.-% liegt.

**Claims**

1. Process for the preparation of aryloxyacetic acids by oxidation of aryloxyethanols of the formula

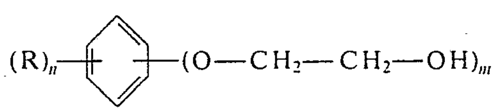

(I)

in which

m represents 1 or 2,
n represents the numeral which results from the difference (6 — m) and
R either individually or independently of one another represent hydrogen, alkyl, cycloalkyl, aryl, aralkyl, alkoxy, cycloalkoxy, aryloxy, hydroxyl, halogen, alkylcarbonyl, arylcarbonyl, carboxyl or nitro, or represent a benzene ring or cycloalkane ring fused to the phenyl ring,

with oxygen or oxygen-containing gases in aqueous alkaline media at temperatures of from 0° C to the boiling point of the reaction mixture in the presence of platinum metal catalysts, characterised in that

the oxidation is carried out in the presence of activators of lead and/or bismuth and/or their compounds and optionally in the additional presence of cadmium and/or its compounds.

2. Process according to Claim 1, characterised in that lead and/or bismuth and optionally additionally cadmium in amounts of $1 \times 10^{-5}$ to $1 \times 10^{-1}$ mol of metal or metal compound per mol of aryloxyethanol to be oxidised are emplyed.

3. Process according to Claim 1, characterised in that lead and/or bismuth and optionally additionally cadmium in amounts of $5 \times 10^{-5}$ to $5 \times 10^{-2}$ mol of metal or metal compound per mol of aryloxyethanol to be oxidised are employed.

4. Process according to Claim 1 to 3, characterised in that platinum and/or palladium are used as platinum metal catalysts.

5. Process according to Claim 1 to 4, characterised in that lead and/or bismuth and optionally additionally cadmium are used in combination with platinum catalysts.

6. Process according to Claim 1 to 4, characterised in that lead and/or bismuth are used in combination with palladium catalysts.

7. Process according to Claim 1 to 6, characterised in that the platinum metal is applied to a support.

8. Process according to Claim 1 to 7, characterised in that active charcoal is used as the support for the platinum metal.

9. Process according to Claim 1 to 8, characterised in that the platinum metal content of the supported catalysts is less than 25% by weight.

10. Process according to Claim 1 to 8, characterised in that the platinum metal content of the supported catalysts is less than 20% by weight when using palladium and less than 5% by weight when using platinum.

**Revendications**

1. Procédé de préparation d'acides aryloxyacétiques par oxydation d'aryloxyéthanols répondant à la formule

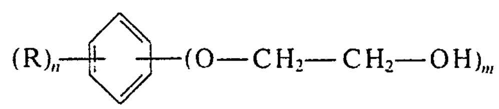 (I)

dans laquelle:

m est égal à 1 ou 2,
n est un nombre égal à la différence (6−m) et
les symboles R représentent individuellement ou indépendamment les uns des autres l'hydrogène, des groupes alkyle, cycloalkyle, aryle, aralkyle, alcoxy, cycloalcoxy, aryloxy, hydroxy, des atomes d'halogènes, des groupes alkylcarbonyle, arylcarbonyle, carboxy, nitro ou un noyau benzénique ou cycloalcane condensé sur le noyau phényle,

par l'oxygène ou des gaz contenant de l'oxygène en milieu aqueux alcalin à des températures comprises entre 0° C et le point d'ébullition du mélange de réaction en présence de catalyseurs à base de métaux de la série du platine, caractérisé en ce que l'oxydation est effectuée en présence d'activateurs consistant en plomb et/ou bismuth et/ou leurs composés et, le cas échéant, en outre en présence de cadmium et/ou de ses composés.

2. Procédé selon la revendication 1, caractérisé en ce que l'on utilise le plomb et/ou le bismuth et, le cas échéant, en outre le cadmium en quantités de $1 \times 10^{-5}$ à $1 \times 10^{-1}$ mole de métal ou de composé métallique par mole d'aryloxyéthanol à oxyder.

3. Procédé selon la revendication 1, caractérisé en ce que l'on utilise le plomb et/ou le bismuth et, le cas échéant, en outre, le cadmium en quantités de $5 \times 10^{-5}$ à $5 \times 10^{-2}$ mole de métal ou de composé métallique par mole d'aryloxyéthanol à oxyder.

4. Procédé selon les revendications 1 à 3, caractérisé en ce que les catalyseurs à base de métaux de la série du platine sont le platine et/ou le palladium.

5. Procédé selon les revendications 1 à 4, caractérisé en ce que l'on utilise le plomb et/ou le bismuth et, le cas échéant, en outre, le cadmium en combinaison avec des catalyseurs au platine.

6. Procédé selon les revendications 1 à 4, caractérisé en ce que l'on utilise le plomb et/ou le bismuth en combinaison avec des catalyseurs au palladium.

7. Procédé selon les revendications 1 à 6, caractérisé en ce que le métal de la série du platine est appliqué sur un support.

8. Procédé selon les revendications 1 à 7, caractérisé en ce que le support du métal de la série du platine est le charbon actif.

9. Procédé selon les revendications 1 à 8, caractérisé en ce que la teneur en métal de la série du platine du catalyseur sur support est inférieure à 25% en poids.

10. Procédé selon les revendications 1 à 8, caractérisé en ce que la teneur en métal de la série du platine des catalyseurs sur support est inférieure à 20% en poids lorsqu'on utilise le palladium et inférieure à 5% en poids lorsqu'on utilise le platine.